## Europäisches Patentamt

## European Patent Office

## Office européen des brevets

(11) Numéro de publication: **0 120 968**
**B1**

# FASCICULE DE BREVET EUROPÉEN

(45) Date de publication du fascicule du brevet:
**29.06.88**

(51) Int. Cl.⁴: **A 01 M 13/00, A 61 L 9/03**

(21) Numéro de dépôt: **83903106.5**

(22) Date de dépôt: **30.09.83**

(86) Numéro de dépôt international:
**PCT/JP 83/00323**

(87) Numéro de publication internationale:
**WO 84/01264 (12.04.84 Gazette 84/10)**

(54) **APPAREIL DE VOLATILISATION THERMIQUE D'UN AGENT MEDICINAL.**

(30) Priorité: 30.09.82 JP 148579/82 U
30.09.82 JP 148580/82 U
30.09.82 JP 148581/82 U
04.10.82 JP 174295/82
06.10.82 JP 177050/82
06.10.82 JP 153100/82 U
08.11.82 JP 169122/82 U
08.11.82 JP 169123/82 U
06.01.83 JP 373/83 U
06.01.83 JP 374/83 U
16.02.83 JP 20971/83 U
26.09.83 JP 177647/83

(43) Date de publication de la demande:
**10.10.84 Bulletin 84/41**

(45) Mention de la délivrance du brevet:
**29.06.88 Bulletin 88/26**

(84) Etats contractants désignés:
**FR**

(56) Documents cité:
**FR-A-2 215 864**
**FR-A-2 530 144**
**GB-A-1 366 041**
**GB-A-2 062 484**
**JP-Y-43 002 047**
**JP-Y-50 006 912**

(73) Titulaire: **DAINIHON JOCHUGIKU CO., LTD., 4-11, Tosabori 1-chome Nishi- ku, Osaka- shi Osaka 550 (JP)**

(72) Inventeur: **KATSUDA, Yoshio, 10- 10, Kamikoutouen 2-chome Nishinomiya- shi, Hyogo 662 (JP)**
Inventeur: **YOSHINAGA, Sadao, 22- 14, Higashisugano 1-chome Ichikawa- shi, Chiba 272 (JP)**
Inventeur: **MASHINE, Hiroyoshi, 5-10, Asahicho 6-chome Souka- shi, Saitama 340 (JP)**

(74) Mandataire: **Tony- Durand, Serge, Cabinet Tony- Durand 77, rue Boissière, F-75116 Paris (FR)**

EP 0 120 968 B1

## Description

La présente invention concerne un fumigateur thermique pour des drogues et produits chimiques, tels que des insecticides, des fongicides, des parfums d'ambiance et des déodorants. Dans le reste de la description le terme "drogue" est utilisé pour désigner, d'une façon générale de tels produits susceptibles de produire, sous l'effet de la chaleur, des fumées ou vapeurs ayant une activité ou propriété déterminée.

Des dispositifs tels que des appareils électriques pour tuer les moustiques, qui effectuent des fumigations à l'intérieur des pièces en chauffant un tampon feutré imprégné de drogues telles que des insecticides, libérant ainsi ces drogues sous forme de fumée à partir du tampon feutré, sont connus dans la technique.

Ainsi le GB-A-1 366 041 illustre un fumigateur de drogues, comportant un boîtier avec un réceptacle à combustible contenant un gaz liquéfié, apte à réagir avec un catalyseur métallique disposé de façon séparée au-dessus du réceptacle à combustible, tandis qu'une plaque est disposée au-dessus du catalyseur métallique pour supporter l'élément insecticide ou drogue à vaporiser. Le boîtier est muni de passages pour l'entrée de l'air ambiant et l'évacuation des gaz de combustion et des vapeurs de drogue. Toutefois, dans l'appareil décrit et représenté, la plaque de support de l'insecticide n'est pas spécialement prévue pour irradier de la chaleur ce qui ne permet pas une vaporisation complète et satisfaisante du produit. En outre, le catalyseur est en contact direct avec la plaque support sur lequel elle repose, ce qui limite le passage d'air ambiant et également rend moins satisfaisantes les conditions dans lesquelles s'effectue la vaporisation de la drogue.

La présente invention concerne un fumigateur pour drogue qui évite ces inconvénients grâce aux dispositions faisant l'objet de la revendication 1.

Plus précisément, le fumigateur pour drogue considéré se caractérise en ce qu'un espace est ménagé entre le catalyseur et le point de sortie des vapeurs ou du gaz combustible hors du réceptacle à combustible et en ce qu'un autre espace est laissé libre entre la plaque et le catalyseur métallique.

L'invention sera plus explicitement exposée à travers deux exemples de réalisation donnés à titre indicatif, en référence aux dessins annexés sur lesquels:

La Figure 1 est une vue en plan représentant un fumigateur thermique typique de la présente invention.

La Figure 2 est une coupe transversale suivant la ligne II-II de la Figure 1.

Les Figures 3(A) et 3(B) sont chacune une vue en perspective d'un élément irradiant de la chaleur typique de la présente invention.

La Figure 4 est une vue en plan d'un fumigateur thermique de la présente invention.

La Figure 5 est une coupe transversale le long de la ligne III-III de la Figure 4.

La Figure 6 est une vue agrandie d'un moyen B de réglage de la soupape dans le fumigateur thermique.

La Figure 7 est une vue agrandie d'un mécanisme A de soupape d'injection du fumigateur thermique de la présente invention.

La Figure 8 est un graphique montrant les résultats obtenus.

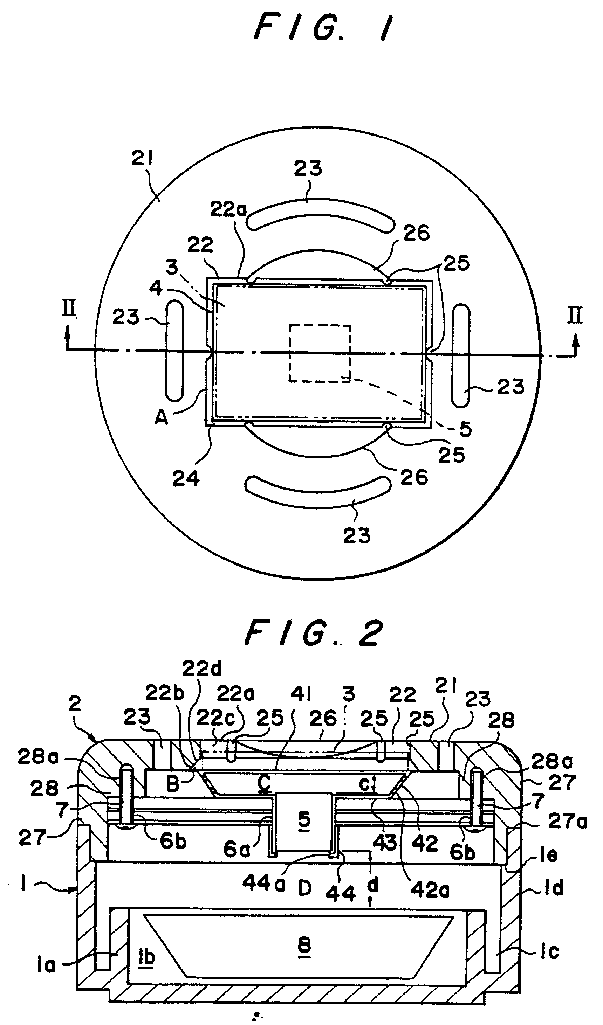

Un fumigateur thermique typique de la présente invention est représenté sur la Figure 1 et sur la Figure 2. Sur les figures, 1 désigne un corps de boîtier ayant la forme d'un tube aveugle, dont l'intérieur est divisé en un réceptacle à combustible 1b et en un réservoir à eau 1c avec une paroi interne cylindrique 1a formée à l'intérieur. Une paroi latérale (paroi externe) 1d du corps de boîtier 1 s'étend vers le haut au-dessus de la paroi interne 1a précitée. Sur le côté interne de l'extrémité supérieure de la partie étendue de la paroi externe 1d, une partie le comportant un redan est formée sur toute la circonférence de celle-ci pour y emboîter un couvercle de boîtier.

Le repère 2 désigne un couvercle de boîtier qui est un élément comprenant une plaque de dessus circulaire 21 et une paroi latérale cylindrique 27 et ayant une coupe transversale pratiquement en forme de "U". La plaque de dessus 21 est munie pratiquement en son centre d'une ouverture rectangulaire 22 et, en dehors des bords de l'ouverture 22, d'orifices d'air 23. L'ouverture 22 sur le côté supérieur 22a de la plaque de dessus a une forme rectangulaire semblable à celle du tampon feutré insecticide 3 qui est mis en place dans le fumigateur thermique, et un peu plus grande. Sur son côté inférieur 22b, elle a une forme rectangulaire semblable à celle de l'extrémité supérieure d'un élément irradiant de la chaleur 4 servant à supporter le tampon feutré insecticide 3, et un peu plus grande. La paroi intérieure définissant l'ouverture 22, par rapport à la direction dans laquelle l'ouverture 22 est percée dans le couvercle du boîtier 2, est divisée par un plan passant au voisinage du milieu de l'épaisseur de la plaque de dessus 21 dans la paroi verticale supérieure 22c et la paroi inférieure 22d inclinée vers le bas. Grâce à un intervalle A (voir figure 1) à former entre le tampon feutré insecticide 3 et la paroi verticale 22c de l'ouverture 22 et à un intervalle B (voir figure 2) à former entre l'élément irradiant de la chaleur 4 et la paroi inclinée 22d de l'ouverture 22, on est assuré d'avoir un espace d'échappement d'air 24 intact servant de libre passage pour l'air entre l'intérieur et l'extérieur du couvercle de boîtier 2, même après que le tampon feutré insecticide 3 a été mis en place sur l'élément irradiant de la chaleur 4. Le repère 25 désigne des protubérances dirigées vers l'intérieur formées sur la paroi de l'ouverture 22. Ces protubérances sont destinées à permettre au tampon feutré insecticide 3 d'être placé d'une manière précise au centre de l'ouverture 22 et à modifier l'écoulement de l'air à travers l'espace d'échappement d'air 24 entre le tampon feutré insecticide 3 et la paroi de l'ouverture 22. Le repère 26 désigne des dépressions dans la surface supérieure de la plaque de dessus 21 et inclinées doucement vers le bas de la périphérie jusqu'au centre de la plaque de dessus 21. Elles se terminent le long des deux côtés principaux de l'ouverture 22. Ces dépressions 26 sont destinées à augmenter la facilité avec laquelle le tampon feutré insecticide 3 est (a) inséré dans l'ouverture 22 et monté sur l'élément irradiant de la chaleur 4 ou (b) retiré du fumigateur thermique.

2

Sur le côté interne de la partie dans laquelle la plaque de dessus 21 et la paroi latérale 27 du couvercle de boîtier 2 se rejoignent, une partie à épaulement 28 est formée le long de la totalité de la circonférence de la paroi latérale 27. Cet épaulement 28 est destiné à tenir solidement un élément de support 6 servant à maintenir en place un catalyseur 5 qui sera décrit complètement décrit ci-après et l'élément irradiant de la chaleur 4 pour recevoir et y retenir le catalyseur. 28a désigne des trous pour les vis devant être utilisées pour fixer les éléments de support. 27a désigne une partie à redan formée le long de la totalité de la périphérie dans la partie inférieure de la paroi latérale 27. Cette partie à redan 27a est formée de façon à s'adapter étroitement dans la partie à redan 1e qui est formée sur la paroi externe 1d du corps de boîtier 1. Le couvercle de boîtier 2 est conçu de façon à recouvrir le côté supérieur du corps de boîtier 1 et à compléter un boîtier entier en permettant à la partie à redan 27a de la paroi latérale de s'adapter confortablement dans la partie à redan 1e de la paroi externe du corps de boîtier 1. Des filetages correspondants peuvent être taillés à l'avance sur les surfaces latérales du boîtier 2 et la paroi interne du corps de boîtier, de façon que le corps de boîtier 1 et le couvercle de boîtier 2 soient assemblés par vissage l'un avec l'autre.

Le tampon feutré insecticide 3 est une plaque rectangulaire de fibres comprimées, imprégnées d'une solution de drogues actives comme insecticide. Cette forme rectangulaire n'est pas déterminante pour que le tampon feutré insecticide 3 remplisse sa fonction. Ce tampon feutré 3 peut avoir une forme quelconque pour autant qu'elle peut être insérée dans l'ouverture 22 du couvercle de boîtier 2.

L'élément irradiant de la chaleur 4 est destiné à supporter le tampon feutré insecticide 3, à lui appliquer de la chaleur et à permettre au tampon feutré 3 de libérer la drogue active comme insecticide en fumée. Cet élément irradiant de la chaleur 4 est constitué d'une plaque irradiant de la chaleur assemblée à une pièce retenant le catalyseur. Une vue en perspective agrandie de cet élément irradiant de la chaleur est donnée dans la figure 3(A).

L'élément irradiant de la chaleur 4 comprend une plaque irradiant de la chaleur horizontale 41 conçue pour porter le tampon feutré insecticide 3 et lui appliquer de la chaleur, les parties latérales 42 s'étendant vers le bas à partir des extrémités opposées de la plaque irradiant de la chaleur 41 et légèrement inclinées l'une vers l'autre, les plaques inférieures 43 s'étendant des extrémités inférieures des parties latérales 42 précitées en direction l'une de l'autre parallèlement à la plaque irradiant de la chaleur 41 précitée, et une pièce retenant le catalyseur 44 formée par une paire d'éléments retenant le catalyseur 441 s'étendant vers le bas à partir des bords opposés des plaques inférieures 43. Les bords opposés 441a de chacun des éléments retenant le catalyseur 441 sont recourbés de façon à dépasser vers le bas en direction du centre de la plaque irradiant de la chaleur et le bord inférieur 441b de ceux-ci est recourbé de manière similaire de façon à dépasser vers l'intérieur en direction du centre. Un catalyseur 5 est maintenu en position par des bords latéraux s'étendant vers l'intérieur 441a et des bords inférieurs 441b. L'élément irradiant de la chaleur 4 fait partie intégrante d'une plaque de métal présentant une excellente conductivité thermique.

Si on le désire, les pièces latérales 42 peuvent être munies de trous 42a pour le passage de l'air à travers lesquels de l'air est apporté au catalyseur et le gaz de combustion sortant du catalyseur 5 sera évacué de l'élément irradiant de la chaleur 4. La plaque irradiant de la chaleur 41 peut être poreuse, de sorte qu'une partie du gaz de combustion sortant du catalyseur monolithique 5 trouvera un passage à travers les pores de la plaque irradiant de la chaleur 41 pour accélérer la réaction d'oxydation du combustible dans le catalyseur monolithique 5 et pour l'empêcher d'interférer avec la convection thermique du gaz de combustion. Le fait de prévoir des pores dans la plaque irradiant de la chaleur 41 n'est pas déterminant pour le fonctionnement de l'élément irradiant de la chaleur 4.

Entre la plaque irradiant de la chaleur 41 et les plaques inférieures 43, il existe un espace C ayant une hauteur fixée par les pièces latérales 42. Grâce à l'espace C ainsi formé, le catalyseur 5 est empêché de venir en contact direct avec la plaque irradiant de la chaleur 41. La distance de l'espace C, à savoir l'intervalle séparant le catalyseur et la plaque irradiant de la chaleur (la pièce destinée à chauffer la drogue) l'un de l'autre (indiquée par C cm sur la figure 2) est avantageusement d'au moins 0,2 cm et mieux encore dans l'intervalle de 0,3 à 3,0 cm. Si le catalyseur et la plaque irradiant de la chaleur sont maintenus en contact mutuel ou s'ils sont séparés par un intervalle trop faible (C < 0,2 cm), ceci fait obstacle à la convection désirée du gaz de combustion et à la réaction d'oxydation du combustible et la plaque irradiant de la chaleur est incapable d'apporter beaucoup de chaleur. C'est seulement lorsque le catalyseur et la plaque supérieure sont séparées l'un de l'autre par un intervalle d'au moins 0,2 cm que la plaque irradiant de la chaleur peut être le plus efficacement chauffée à la température élevée désirée par la convection du gaz de combustion sortant du catalyseur.

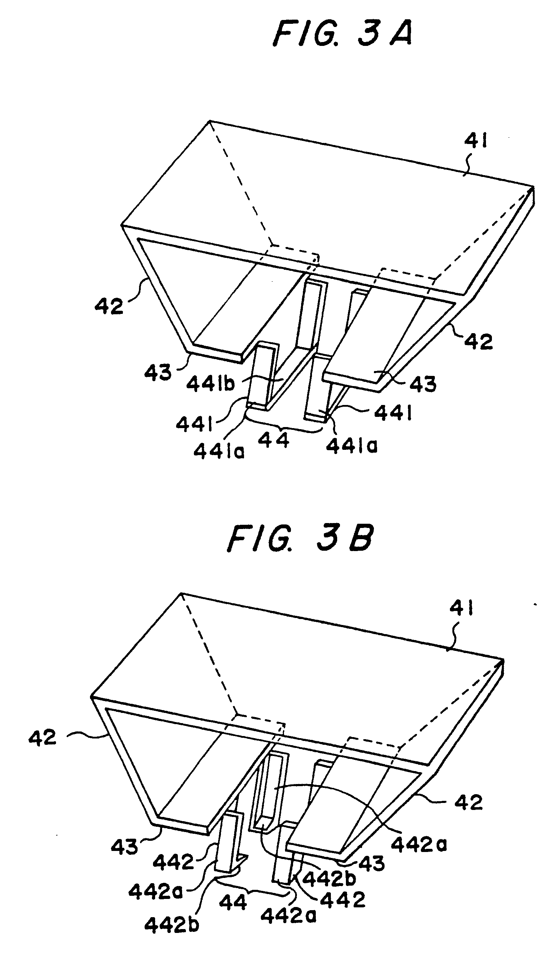

Une variante de l'élément irradiant de la chaleur est représentée sur la figure 3 (B). Tout en ayant pratiquement la même construction que la plaque irradiant de la chaleur décrite jusqu'à présent, cet élément irradiant de la chaleur modifié comprend une plaque irradiant de la chaleur 41, des plaques latérales 42, des pièces inférieures 43 et quatre pieds minces 442 s'étendant vers le bas à partir des bords internes des pièces inférieures 42. Chacun des pieds 442 comprend une mince paroi latérale 442a ayant une section transversale en forme de L et un fond 442b horizontal s'étendant vers l'intérieur à partir de l'extrémité inférieure de la paroi latérale 442a et conçu pour porter le catalyseur en position. Deux de ces pieds 442 sont fixés opposés l'un à l'autre dans la position indiquée pour chacune des pièces inférieures 43 précitées. L'ensemble des quatre pieds 442 constitue collectivement une pièce retenant le catalyseur 44.

Cet élément irradiant de la chaleur modifié présente l'avantage que la conduction de chaleur du catalyseur 5 à la plaque irradiant de la chaleur 41 s'effectue sans aucun obstacle et que l'élément irradiant de la chaleur 4

3

lui-même bénéficie d'une réduction de poids car la pièce retenant le catalyseur 44 est formé de quatre pieds minces 442.

Le catalyseur monolithique 5 a une forme angulaire permettant une insertion confortable dans la partie formant pilier angulaire 44 de la plaque irradiant de la chaleur 4 précitée. Il comprend un support céramique de structure en nids d'abeille et un métal catalytiquement actif tel que du platine ou du palladium déposé sur le support céramique. Le catalyseur 5 à utiliser dans la présente invention n'est évidemment pas limité à cette description. Il peut être sous forme d'un agrégat de perles ou d'une masse de laine, etc. suivant le cas.

6 désigne un élément de support servant à fixer l'élément irradiant de la chaleur 4 et le catalyseur 5 sur le couvercle de boîtier 2. Il s'agit d'une plaque circulaire ayant un diamètre tel que sa périphérie puisse buter intimement contre la surface interne de la paroi latérale 27 du couvercle 2. L'élément de support 6 est muni, au voisinage de son centre, d'une pièce creuse 6a capable de recevoir l'insertion de la partie de pilier angulaire 44 de l'élément irradiant de la chaleur 4 précité. L'élément de support 6 est en outre percé en des positions appropriées de trous 6b pour l'insertion de vis 7 qui sont fixées au couvercle 2. Les trous 6b pour l'insertion de ces vis sont percés en alignement précis avec les trous de vis 28a qui sont formés dans l'épaulement 28 du couvercle 2. Bien que la matière de l'élément de support 6 ne soit pas particulièrement limitée, elle est soit imperméable à l'air, soit légèrement perméable à l'air et il est souhaitable qu'elle résiste à la chaleur et empêche le passage de chaleur. Elle est avantageusement formée de fibres de verre, par exemple.

Le repère 8 désigne un récipient à combustible qui est monté dans le réceptacle à combustible 1b. Il est ouvert sur le côté supérieur et conçu pour être rempli intérieurement d'un combustible volatil. Le récipient à combustible 8 rempli de combustible est mis en place dans le réceptacle à combustible 1b du corps de boîtier 1. Entre le récipient à carburant 8 et le catalyseur 5, est maintenu un espace D de hauteur déterminée.

Le combustible à utiliser dans le fumigateur thermique représenté sur les figures 1 et 2 doit être volatil et amorcer une réaction exothermique avec le catalyseur ci-dessus. Comme exemples de combustibles satisfaisant à cette exigence, on citera les alcools. Parmi les alcools, on préfère le méthanol ou l'éthanol. Des formes concrètes sous lesquelles ces alcools sont effectivement utilisables dans l'invention sont des alcools liquides, des polymères carboxyvinyliques, des copolymères de l'anhydride maléique avec l'isobutylène, des copolymères de l'alcool vinylique avec l'acide acrylique, des alcools gélifiés avec des dérivés de l'amidon, et un combustible solide ayant des alcools comme constituants principaux.

Dans le fumigateur thermique décrit ci-dessus, en se référant aux figures 1 et 2, le tampon feutré insecticide 3 est inséré dans l'ouverture 22 du couvercle de boîtier 2 et mis en position sur la plaque irradiant de la chaleur 41 de l'élément irradiant de la chaleur 4. Le carburant alcoolique montant en fumée du récipient à carburant 8 remplit l'espace D, puis monte à travers le catalyseur 5 disposé au-dessus du récipient à combustible 8. Au cours de la traversée du catalyseur, le combustible alcoolique est oxydé par le catalyseur de platine ou de palladium. Le gaz de combustion obtenu est évacué du catalyseur 5. Le gaz de combustion continue à monter ; il remplit l'espace C, passe à travers les orifices à air 42a, s'écoule à travers l'intervalle B et l'intervalle A et passe dans l'air ambiant. La chaleur de réaction produite par l'oxydation du combustible alcoolique sur le catalyseur 5 est transmise par le courant de convection du gaz de combustion à la plaque irradiant de la chaleur 41 de l'élément irradiant de la chaleur 4 situé au-dessus du catalyseur 5, provoquant une élévation de la température de la plaque 41. La chaleur de réaction prenant naissance dans le catalyseur est transmise séparément par conduction des pièces inférieures 43, à travers les plaques latérales 42, à la plaque irradiant de la chaleur 41. Ainsi, la température de la plaque irradiant de la chaleur 41 est uniformément augmentée. De ce fait, le tampon feutré insecticide 3 monté sur celle-ci est uniformément chauffé. En conséquence, le constituant actif du point de vue insecticide contenu dans le tampon feutré insecticide 3 est diffusé en fumées dans l'air ambiant par l'ouverture 24 du couvercle de boîtier 2. Pendant ce temps, l'air indispensable à la réaction d'oxydation du carburant alcoolique dans le catalyseur 5 pénètre à travers les orifices à air 23 percés dans la plaque de dessus 21 du couvercle de boîtier 2, il traverse les orifices à air 42a des plaques latérales 42 et il atteint sa destination, le catalyseur 5. Une partie de l'air ainsi nécessaire pour la réaction d'oxydation dans le catalyseur 5 est apportée séparément à travers les intervalles A et B au catalyseur 5. L'eau résultant de la réaction d'oxydation du combustible est recueillie et stockée dans le réservoir à eau 1c du corps de boîtier 1.

On décrira à présent le fonctionnement d'un fumigateur thermique de la présente invention modifié pour le cas de l'utilisation d'un gaz liquéfié comme combustible. Ce fumigateur est caractérisé en ce qu'il comprend dans un boîtier un récipient pour y renfermer de façon étanche un gaz liquéfié, une buse communiquant avec le récipient ci-dessus par une soupape, un catalyseur métallique disposé dans une position dans laquelle le gaz sortant de la buse frappe le catalyseur métallique, et une pièce irradiant de la chaleur disposée au voisinage du catalyseur et conçue pour fournir de la chaleur pour la vaporisation de la drogue, et en ce qu'il comporte également, dans le boîtier ci-dessus, un passage pour une arrivée d'air et/ou pour l'évacuation du gaz de combustion et un système de régulation pour réguler l'ouverture et la fermeture de la soupape ci-dessus.

Dans un des aspects de cette variante, le catalyseur métallique peut être disposé au-dessus de la buse à travers un espace d'une certaine hauteur et l'élément irradiant de la chaleur pour la fumigation thermique de la drogue peut être disposé au-dessus du catalyseur métallique à travers un espace d'une certaine hauteur.

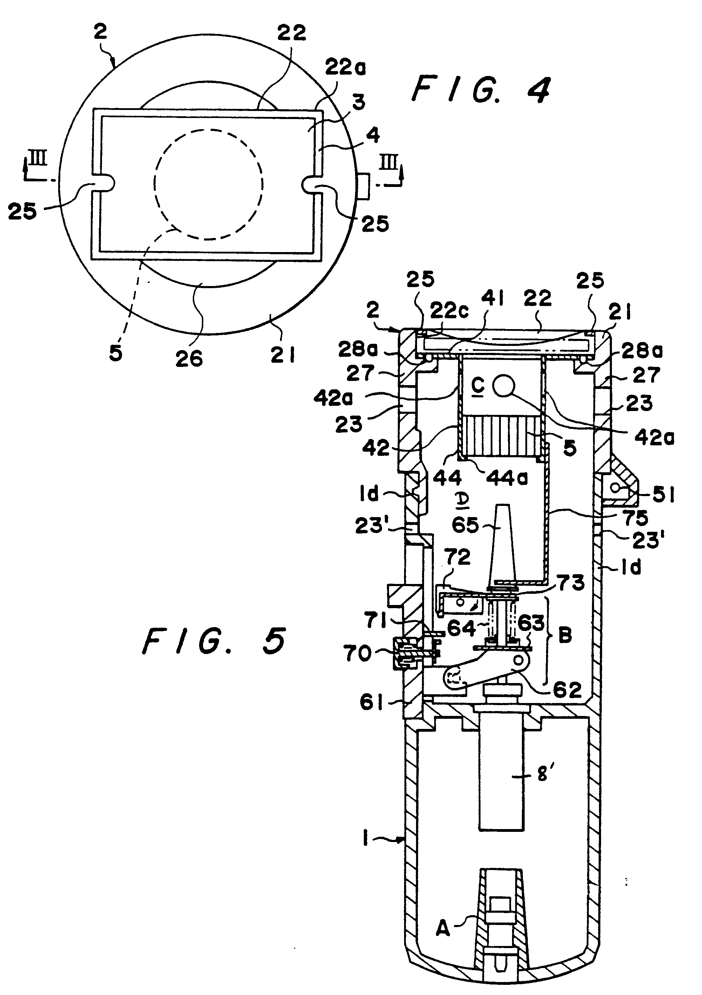

La figure 4 est une vue en plan représentant un fumigateur thermique typique conforme à la variante décrite ci-dessus et la figure 5 est une coupe transversale suivant la ligne III-III de la figure 4. Sur les figures 4 et 5, le repère 1 désigne un boîtier tubulaire aveugle renfermant un récipient 8' qui est muni d'un mécanisme de soupape d'injection A pour enfermer hermétiquement un gaz liquéfié dans le récipient. Le récipient 8' est séparé du reste du corps de boîtier 1 dans la partie médiane du corps de boîtier 1. Le corps de boîtier 1 est en

4

outre muni d'orifices à air 23'.

Le repère 2 désigne un couvercle de boîtier. Dans le présent mode de réalisation, ce couvercle de boîtier 2 est un élément dont la section transversale a pratiquement la forme de la lettre "U"; il comprend une plaque de dessus circulaire 21 et une paroi latérale tubulaire 27. L'ouverture 22 sur le côté frontal 22a de la plaque de dessus a une forme rectangulaire similaire à celle du tampon feutré insecticide 3 disposé dans le fumigateur thermique, et un peu plus grande, et également similaire au côté supérieur de l'élément irradiant de la chaleur 4 servant à supporter le tampon feutré insecticide 3. La paroi définissant l'ouverture 22, se rapportant à la direction dans laquelle l'ouverture 22 est creusée dans la plaque de dessus, comprend une surface verticale 22c parallèle à la direction de l'épaisseur de la plaque de dessus 21. Le repère 25 désigne des protubérances formées sur la paroi verticale 22c de l'ouverture faisant saillie vers l'intérieur à l'intérieur de l'ouverture 22. Ces protubérances sont destinées à permettre une mise en place précise du tampon feutré insecticide 3 au centre de l'ouverture 22 et à l'empêcher de tomber au hasard dans une autre position. Le repère 26 désigne des dépressions dans la surface de la plaque de dessus 21 légèrement inclinées vers le bas de la périphérie au centre de la plaque de dessus et se terminant le long des deux côtés principaux de l'ouverture 22. Ces dépressions 26 sont destinées à augmenter la facilité avec laquelle le tampon feutré insecticide 3 est inséré dans l'ouverture 22 et monté sur l'élément irradiant de la chaleur 4 ou retirée du fumigateur thermique.

La paroi latérale tubulaire 27 est munie d'orifices à air 23 qui permettent un libre écoulement de l'air et une libre évacuation des gaz de combustion.

Le couvercle de boîtier 2 est monté sur le corps de boîtier 1 au moyen d'un pivot 51 formé sur la paroi latérale 1d du corps de boîtier à la partie inférieure de la paroi latérale 27 du couvercle de boîtier et il peut être ouvert et fermé librement par rapport au corps de boîtier 1.

Sur le côté arrière de la partie où la plaque de dessus 21 et la plaque latérale 27 du couvercle de boîtier 2 se réunissent, une partie en épaulement 28 est formée le long de la périphérie entière de la paroi latérale 27. Cette partie en épaulement 28 est destinée à maintenir en position le catalyseur 5 (qui sera décrit plus complètement ci-dessous) et l'élément irradiant de la chaleur 4 recevant et retenant le catalyseur 5. Le repère 28a désigne des trous pour l'insertion de vis servant à fixer l'élément irradiant de la chaleur 4.

Le repère 3 désigne un tampon feutré insecticide qui est une plaque rectangulaire de fibres comprimées imprégnées d'une solution de constituants actifs du point de vue insecticide. Cette forme rectangulaire n'est pas déterminante pour la fonction que doit remplir le tampon feutré insecticide. Ce tampon feutré 3 peut avoir n'importe quelle forme désirée dans la mesure où elle peut être insérée dans l'ouverture 22 du couvercle de boîtier 2.

Le repère 4 désigne un élément irradiant de la chaleur dont le rôle est de supporter le tampon feutré insecticide 3 et de lui appliquer de la chaleur pour que le tampon feutré 3 puisse libérer le composant insecticide en fumées. L'élément irradiant de la chaleur 4 comprend une plaque irradiant de la chaleur 41 conçue pour porter le tampon feutré insecticide 3 et lui appliquer de la chaleur, une pièce tubulaire latérale 42 s'étendant vers le bas pratiquement à partir du centre de la plaque irradiant de la chaleur 41, et un élément tubulaire 44 s'étendant encore vers le bas à partir de l'élément tubulaire latéral et conçu pour retenir le catalyseur. Tous les éléments constitutifs de l'élément irradiant de la chaleur 4 sont en métal. L'élément tubulaire latéral 42 est muni de trous de passage d'air 42a à travers lesquels le gaz de combustion sortant du catalyseur peut être évacué dans l'air ambiant. La plaque irradiant de la chaleur 41 peut être poreuse. Les pores de la plaque irradiant de la chaleur 41 permettront l'échappement d'une partie du gaz de combustion sortant du catalyseur monolithique 5. Ils servent avantageusement de moyens pour favoriser la réaction d'oxydation du carburant dans le catalyseur monolithique 5 et pour permettre à la convection du gaz de combustion de s'effectuer sans obstacle. L'existence de ces pores n'est pas déterminante pour le fonctionnement de la plaque irradiant de la chaleur. L'élément tubulaire 44 est muni à son extrémité inférieure d'un collet 44a faisant saillie vers l'intérieur de l'élément tubulaire 44, de sorte que lorsque le catalyseur monolithique 5 est mis en place dans cet élément tubulaire 44, il peut être retenu de manière sûre sur le collet 44a. Entre l'élément irradiant de la chaleur 4 et le catalyseur 5, est réservé un espace C ayant une hauteur déterminée. Cet espace C sert à garder le catalyseur monolithique 5 séparé de la plaque irradiant de la chaleur 41 supportant le tampon feutré insecticide 3.

Le catalyseur monolithique 5 est d'une forme tubulaire adaptée pour l'insertion dans l'élément tubulaire 44 de l'élément irradiant de la chaleur 4 précité. Il comprend un support céramique de structure en nids d'abeille et un métal catalytiquement actif, tel que du platine ou du palladium déposé sur le support de céramique. Le catalyseur 5 à utiliser dans la présente invention n'est pas limité à cette forme particulière. Il peut revêtir la forme d'un agrégat de perles ou être en masse ou sous forme de tampon, par exemple.

Il est avantageux d'isoler l'espace au-dessus de la buse de l'espace au-dessus du métal du catalyseur métallique avec une plaque thermiquement isolante pour séparer les orifices d'alimentation en air et les orifices d'évacuation du gaz de combustion et utiliser en même temps cette plaque d'isolation thermique comme élément retenant le catalyseur.

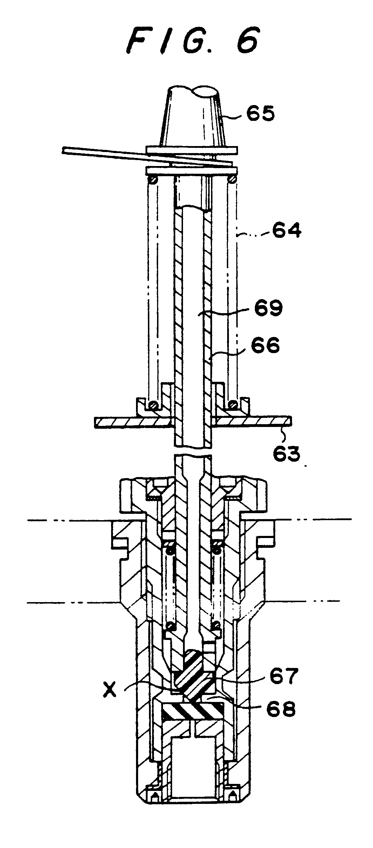

Le repère 8' désigne un récipient pour contenir de manière étanche un gaz liquéfié. Le récipient est muni d'une buse 65 qui communique avec l'intérieur du récipient par une soupape (figure 6). Ce récipient 8' est rempli d'un gaz liquéfié qui sert de combustible.

Comme combustible on peut utiliser n'importe quel gaz liquéfié dans la mesure où le gaz est capable de provoquer une réaction d'oxydation à l'aide du catalyseur précité. Comme exemples concrets de gaz liquéfié satisfaisant à cette exigence, on citera le gaz de pétrole liquéfié (LPG), l'éther diméthylique, l'hexane, le benzène, et des gaz utilisables dans des briquets. Le récipient 8' est muni de moyens de réglage de la soupape B capables de régler l'ouverture et la fermeture de la soupape x servant à ajuster la libération du gaz liquéfié. Ce moyen de régulation B est enclenché avec un commutateur 61 qui est prévu dans le corps du boîtier de façon à régler l'apport de gaz liquéfié au milieu du catalyseur 5 pour uniformiser ainsi la température de l'élément irradiant la chaleur 4.

Dans le fumigateur thermique construit comme il a été décrit ci-dessus, le tampon feutré insecticide 3 est inséré dans l'ouverture 22 du couvercle de boîtier 2 puis mis en place sur la plaque irradiant de la chaleur 41 de l'élément irradiant de la chaleur 4. Le combustible de gaz liquéfié sortant du récipient 8' et traversant la buse et l'air admis par les orifices à air 23, 23' passent ensemble à travers le catalyseur 5 disposé au-dessus du récipient 8'. Dans le catalyseur 5, le carburant de gaz liquéfié est oxydé à travers les catalyseurs de platine ou de palladium et le gaz de combustion obtenu est évacué du catalyseur 5. Ce gaz de combustion monte encore pour remplir l'espace C, il traverse les orifices à air 42a pratiqués dans l'élément tubulaire latéral 42 de l' élément irradiant de la chaleur 4, et passe dans l'air ambiant à travers les orifices à air 23. La chaleur de réaction produite sous l'effet de l'oxydation du carburant de gaz liquéfié dans le catalyseur 5 est transférée par convection à la plaque irradiant de la chaleur 41 de l'élément irradiant de la chaleur 4 disposé au-dessus du catalyseur, provoquant une élévation de la température de la plaque irradiant de la chaleur 41. La chaleur de la réaction produite à l'intérieur du catalyseur 5 est également transférée par conduction à travers un élément tubulaire latéral 42 de l'élément irradiant de la chaleur 4 constitué d'une matière métallique vers la plaque irradiant de la chaleur 41. Par conséquent, la température de la plaque irradiant de la chaleur 41 est uniformisée. La température de la plaque irradiant de la chaleur 41 étant ainsi augmentée, le tampon feutré insecticide 3 monté sur la plaque irradiant de la chaleur 41 est chauffé uniformément à un degré suffisant pour que le constituant actif du point de vue insecticide contenu dans le tampon feutré insecticide 3 soit dispersé en fumées dans l'air ambiant à travers l'ouverture 22 du couvercle de boîtier 2. En même temps, l'air nécessaire pour la réaction d'oxydation du carburant de gaz liquéfié dans le catalyseur est admis à travers les orifices à air 23 formés dans la plaque de dessus 21 du couvercle de boîtier 2 et les orifices à air 23' du corps de boîtier et il est envoyé au catalyseur 5.

On décrira à présent le fonctionnement du système de réglage de la soupape lors du fonctionnement réel du fumigateur thermique ayant la construction indiquée ci-dessus.

La figure 6 est une vue agrandie du système de réglage de la soupape B pour l'utilisation dans le fumigateur thermique de la présente invention. En premier lieu, un bouchon 70 est retiré, puis un interrupteur 61 est poussé vers le haut dans la position d'ouverture de la soupape (pour la libération du gaz). En conséquence, une plaque basculante 62 est manoeuvrée pour élever un support métallique 63 et le support métallique 63 ainsi élevé comprime un ressort 64. La force dirigée vers le haut ainsi exercée par le ressort soulève un corps de soupape mobile 66 qui est muni d'une buse 65.

Le mouvement vers le haut du corps de soupape mobile 66 provoque la séparation d'un élément de soupape 67 d'un siège de soupape stationnaire 68 et par conséquent ouvre la soupape x qui est composée de l'élément de soupape 67 et du siège de soupape stationnaire 68. Cette ouverture de la soupape x permet au gaz liquéfié de traverser un trou de guidage 69 formé dans le corps de soupape mobile puis d'être libéré à travers la buse 65.

A ce moment, le couvercle de boîtier 2 est ouvert pour découvrir la buse 65 et le gaz liquéfié sortant de la buse 65 est allumé avec une allumette ou un briquet. Puis, le couvercle de boîtier 2 est remis en place de manière étanche sur le corps de boîtier 1 et la combustion du gaz liquéfié est poursuivie pendant plusieurs secondes jusqu'à 10 secondes environ, jusqu'à ce que la température du catalyseur soit élevée à la valeur prescrite.

A ce stade, le bouchon 70 est poussé pour être libéré et l'interrupteur est poussé plus loin vers le haut.

Une plaque de relevage 71 dépassant de l'interrupteur 61 fait alors tourner une plaque rotative 72 dans la direction de la flèche et communique un mouvement vers le bas à l'extrémité d'un dispositif d'abaissement 73 faisant partie intégrante de la plaque rotative 72. La buse 65, qui engrène avec l'extrémité du dispositif d'abaissement 73, est déplacée vers le bas, amenant l'élément de soupape 68 en contact solide avec le siège de la soupape stationnaire 68, fermant ainsi la soupape x.

Lors de cette fermeture de la soupape x, la libération du gaz liquéfié par la buse 65 est arrêtée et la flamme du gaz liquéfié est éteinte. Ensuite, l'interrupteur 61 est déplacé vers le bas et le bouchon 70 est bloqué en position d'ouverture de la soupape. Par ce blocage du bouchon dans la position d'ouverture de la soupape, la soupape x est ouverte à nouveau et la libération du gaz par la buse 65 est maintenue en continu.

Comme le catalyseur 5 est à présent retenu dans son état chauffé prescrit, le gaz liquéfié libéré par la buse 65 est amené à réagir rapidement et le catalyseur est maintenu chauffé.

Pour empêcher maintenant une surchauffe possible du catalyseur 5, on actionne alors un bimétal 75 pour régler le volume d'écoulement du gaz liquéfié à travers la soupape x. Plus précisément, si le corps de soupape mobile 66 est laissé sous tension élastique, il est maintenu poussé vers le haut dans la position indiquée sur la figure. Ainsi, le bimétal 75 est construit de telle sorte que son extrémité libre puisse abaisser la buse contre la force d'excitation. L'importance de l'abaissment de la buse 65, c'est-à-dire l'importance du déplacement du corps de soupape mobile 65, dépend de la température du catalyseur par l'intermédiaire de ce bimétal. En conséquence, le corps de soupape 67 et le siège de soupape stationnaire 68 sont amenés par la force d'abaissement du bimétal 75 à faire varier le degré de fermeture de la soupape.

En conséquence, la quantité de gaz liquéfié à libérer à travers la buse 65 est réglée automatiquement par la température du catalyseur 5, avec cette conséquence naturelle que la température de réaction dans le catalyseur 5 est maintenue à une valeur déterminée. Puis, la chaleur de réaction formée dans le catalyseur 5 maintient effectivement la température de la plaque irradiant de la chaleur 41 de l'élément irradiant de la chaleur 4 un niveau uniforme comme il a été décrit ci-dessus et le tampon feutré insecticide 3 monté sur la plaque irradiant de la chaleur 41 est chauffé uniformément pour que le constituant actif du produit insecticide soit libéré en fumées de la nappe feutrée 3.

On met fin à l'utilisation du fumigateur thermique de la présente invention en relâchant la butée puis en déplaçant le commutateur 61 vers le bas dans la position de fermeture de la soupape. Ce mouvement vers le bas de l'interrupteur 61 provoque une rotation de la plaque basculante 62 dans le sens inverse des aiguilles d'une montre et par conséquent le support métallique 63 engagé dans la plaque basculante 62 est déplacé vers le bas. Par ce mouvement vers le bas du support métallique, la force d'excitation du ressort 64 exercée dans la direction du soulèvement de la buse 65 est affaiblie. En conséquence, la buse 65 peut se déplacer vers le bas et l'élément de soupape 67 est amené à s'appliquer sur le siège de soupape stationnaire 68. La soupape x est ainsi fermée.

Dans les modes de réalisation décrits jusqu'à présent le récipient pour renfermer hermétiquement un gaz liquéfié est invariablement incorporé aux corps de boîtiers respectifs 1 et est muni d'un mécanisme de soupape d'injection A (figure 5) capable d'introduire librement le gaz liquéfié dans le récipient 8.

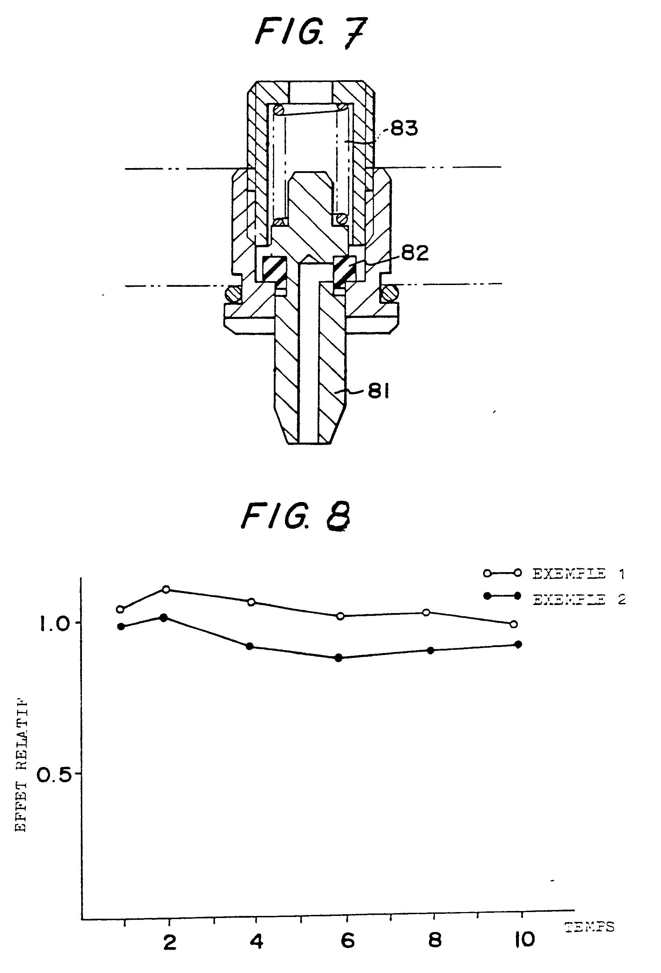

Une vue agrandie du mécanisme de soupape d'injection A est représentée sur la figure 7.

Ce mécanisme de soupape d'injection A est similaire dans son principe de fonctionnement aux mécanismes de soupapes d'injection que l'on trouve habituellement dans les briquets à gaz. En particulier, un élément mobile 81 est fermé avec un joint étanche 82 par l'entremise de la force d'excitation exercée par un ressort 83. Lors de l'injection du gaz liquéfié, l'élément mobile 81 est déplacé vers le haut (par rapport à la figure 9), et par conséquent, le joint 82 est également déplacé vers le haut, donnant naissance à un orifice d'injection (soupape ouverte). Le gaz liquéfié est ainsi injecté à travers la soupape ouverte dans le récipient.

Le récipient 8 destiné à renfermer hermétiquement le gaz liquéfié n'est pas limité à celui qui est incorporé dans le corps de boîtier 1. Si on le désire, un récipient à gaz liquéfié du type cartouche ressemblant à une bouteille de gaz peut être formé séparément du corps de boîtier 1 et utilisé indépendamment du corps de boîtier 1.

L'insecticide qui peut être utilisé par le fumigateur thermique de la présente invention peut être n'importe lequel des divers agents insecticides adoptés jusqu'à présent pour être utilisés par exemple dans des appareils de destruction des moustiques. Comme exemples typiques de ces insecticides on citera des insecticides pyréthroïdes tels que le dl-cis/trans-chrysanthémate de 3-allyl-2-méthylcyclopenta-2-ène-4-one-1-yle, le d-cis/trans-chrysanthemate de 3-allyl-2-méthylcyclopenta-2-ène-4-one-1-yle, le d-trans-chrysanthémate de d-3-allyl-2-méthyl-cyclopenta-2-ène-4-one-1-yle, le d-cis/trans-chrysanthémate de 5-propargyl-2-furylméthyle, le d-cis/trans-chrysanthémate de 1-éthynyl-2-méthylpenta-2-ène-1-yle et le 2,2,3,3-tétraméthyl cyclopropane carboxylate de 1-éthynyl-2-méthylpenta-2-ène-1-yle. En outre, le butoxyde de pipéronyle, le N-(2-éthylhexyl)-1-isopropyl-4-méthylbicyclo-[2,2,2]-octo-5-ène-2,3-dicarboxy imide, et l'éther octachlorodipropylique sont des exemples typiques de l'adjuvant pyréthroïde qui peut être utilisé en combinaison avec l'agent insecticide précité. Ces constituants insecticides sont incorporés par imprégnation d'un tampon feutré de fibres comprimées. Le tampon feutré peut en outre comprendre, outre les constituants insecticides précités, un antioxydant tel que BHT, BHA, ou DBH, capable de servir de stabilisant pour le constituant insecticide, des colorants qui, par exposition à la chaleur, changent de couleur, et indiquent ainsi si le tampon feutré a déjà été utilisé ou non, et des parfums.

Dans le fumigateur thermique de la présente invention, un récipient d'aluminium rempli d'un produit chimique solide capable d'être vaporisé en fumées convenant pour la fumigation visée par la présente invention peut être utilisé à la place du tampon feutré imprégné d'une solution de constituants actifs du point de vue insecticide. Si nécessaire, l'insecticide peut être remplacé par un agent fongicide, un parfum d'ambiance ou un désinfectant. N'importe lequel des divers agents fongicides qui possèdent la volatilité d'alcools ou de dioxines peut être utilisé à cet effet.

Le fumigateur thermique de la présente invention n'est pas limité aux constructions représentées sur les figures 1 à 6. Par exemple, les orifices à air sont indispensables à l'apport d'air nécessaire pour la réaction d'oxydation catalytique du carburant et pour la libération du gaz de combustion résultant de la réaction d'oxydation de l'intérieur du corps de boîtier dans l'air ambiant. Si nécessaire, ils peuvent être formés dans la paroi latérale du couvercle du boîtier ou dans la paroi externe du corps de boîtier à d'autres endroits que ceux représentés sur ces figures. Lorsque les orifices à air pour l'apport d'air sont formés dans la paroi externe du corps de boîtier, il est nécessaire de les former dans la partie la plus basse possible de la paroi externe du corps de boîtier ou en bas du corps de boîtier pour éviter que le combustible volatilisé ne passe à travers les orifices à air. Si on le désire, un orifice à air peut être utilisé en même temps pour l'apport d'air et pour la libération du gaz de combustion. Dans ce cas, l'espace formé entre le tampon feutré insecticide et l'ouverture peut être utilisé comme orifice à air et aucun autre orifice à air n'est nécessaire.

La forme de la plaque irradiant de la chaleur n'est pas limitée à l'une ou l'autre des formes représentées sur les figures. De même, la forme du catalyseur n'est pas limitée à l'une ou l'autre des formes représentées sur les figures. Par exemple, en vue de protéger le catalyseur contre l'adhérence de la drogue libérée par le tampon feutré , il est avantageux d'éliminer l'intervalle formé autour de la plaque irradiant de la chaleur pour libérer de l'air et de disposer une ouverture pour l'évacuation d'air à un niveau très inférieur à celui de la plaque irradiant de la chaleur.

Il est admissible de disposer le catalyseur dans un élément réceptacle formé séparément de la plaque irradiant de la chaleur de forme plane indiquée ci-dessus et maintenue en place par transpercement à travers l'élément de support. Dans ce cas, l'élément réceptacle peut comprendre des pièces de maintien verticalement opposées conçues pour pincer entre elles le catalyseur. L'élément latéral peut être omis lorsque l'élément réceptacle du catalyseur est formé séparément de la plaque irradiant de la chaleur comme il a été décrit ci-dessus. Il est néanmoins nécessaire que l'espace C d'une hauteur fixée soit interposé entre la plaque irradiant de la chaleur et le catalyseur. La hauteur de cet espace C, à savoir l'intervalle séparant le catalyseur et la plaque irradiant de la chaleur l'un de l'autre (indiqué par C cm sur la figure 2) doit être d'au moins 0,2 cm et de préférence dans l'intervalle de 0,3 à 3,0 cm. Si le catalyseur et la plaque irradiant de la chaleur sont maintenus en contact intime (C = 0 cm) ou sont séparés par une distance faible (C < 0,2 cm), on n'obtient pas un chauffage abondant de la plaque irradiant de la chaleur parce que la convection du gaz de convection et la réaction d'oxydation sont empêchées de s'effectuer régulièrement dans cet intervalle. C'est seulement lorsque le catalyseur et la plaque irradiant de la chaleur sont séparés l'un de l'autre par une distance d'au moins 0,2 cm que la plaque irradiant de la chaleur peut être le plus efficacement chauffée à la température élevée par la convection de la chaleur du gaz de combustion émanant du catalyseur.

La forme du catalyseur n'est limitée à aucune forme représentée sur les figures. Le catalyseur peut être un catalyseur monolithique ayant la forme d'un cylindre.

Le récipient rempli du combustible fonctionne efficacement dans le fumigateur thermique de la présente invention pour autant qu'il a une ouverture à sa partie supérieure et que le combustible qui est contenu est efficacement vaporisé par le fumigateur.

Pour empêcher le retour de l'eau formée au cours de la réaction d'oxydation dans le récipient à combustible, on peut prévoir à l'intérieur du fumigateur un réceptacle à eau ayant la forme d'un entonnoir. Sinon, la partie du récipient à combustible située au-dessus du col de celui-ci peut être évasée vers le haut pour donner naissance à un réceptacle à eau ayant la forme d'un entonnoir.

Lorsqu'on adopte un carburant sous forme gélifiée ou un carburant solide, la pratique consistant à utiliser le carburant enveloppé dans un tissu grossier tel qu'une gaze ou un non-tissé, une matière expansée ou une céramique poreuse ou une matière plastique, ou une autre matière qui ne constitue pas un obstacle à la vaporisation de la drogue, se révèle avantageuse pour éviter que le combustible ne fuie hors du récipient ou pour ajuster la quantité du combustible à vaporiser.

La hauteur de l'espace D, c'est-à-dire la distance d séparant le combustible et le catalyseur l'un de l'autre, est avantageusement d'au moins 0,3 cm, et de préférence dans l'intervalle de 0,5 à 10,0 cm. Si le combustible et le catalyseur sont maintenus en contact intime l'un avec l'autre (d = 0 cm), ou s'ils sont séparés l'un de l'autre par une distance trop faible (d < 0,3 cm), il en résulte cet inconvénient que le combustible volatilisé ne s'écoule pas efficacement dans le catalyseur et que du combustible est gaspillé. C'est seulement lorsque le combustible et le catalyseur sont séparés l'un de l'autre par une distance d'au moins 0,3 cm que le combustible volatilisé peut traverser le catalyseur de manière régulière et que la réaction d'oxydation s'effectue avantageusement.

Lorsqu'on adopte un carburant solide volatil, ou un carburant liquide volatil, on peut construire un réceptacle à combustible pour recevoir et retenir le combustible de façon à servir de récipient susceptible d'être rechargé avec une nouvelle alimentation et il peut être utilisé à la place d'un récipient spécifiquement conçu pour contenir le combustible.

On décrira à présent le fumigateur thermique de la présente invention en se référant aux exemples de travail.

## Exemple 1

On dilue 5 g de d-cis/trans-chrysanthémate de 5-propargyl-2-furylméthyle, 15 g de N-(2-éthylhexyl)-1-isopropyl-4-méthyl-bicyclo[2,2,2]oct-5-ène-2,3-dicarboxy imide, 1,5 g de DBH, et 0,2 g de 1,4-diisopropyl aminoanthraquinone à un volume total de 100 ml avec de l'acétone. On imprègne une plaque de fibres comprimées de 35 x 22 x 2,8 mm avec 1 ml de la solution obtenue. La plaque mouillée est séchée dans un courant d'air pour produire un tampon feutré insecticide. Pour l'utilisation, ce tampon feutré insecticide est monté sur la plaque irradiant de la chaleur du fumigateur thermique représenté sur les figures 1 et 2.

## Exemple 2

On dissout 6 g de d-cis/trans-chrysanthémate de 3-allyl-2-méthylcyclopenta-2-ène-4-one-1-yle, 4 g de pipéronyle butoxyde, 2 g de BHT et 0,3 g de 1,4-diméthyl aminoanthraquinone jusqu'à un volume total de 100 ml avec de l'acétone. On imprègne de 1 ml de la solution obtenue la même plaque de fibres comprimées que celle de l'exemple 1 et on la traite de la même façon pour produire un tampon feutré insecticide. Pour l'utilisation, ce tampon feutré est monté sur la plaque irradiant de la chaleur du fumigateur thermique représenté sur les figures 1 et 2.

## Exemple 3

On prépare une solution en mélangeant 10 g de d-cis/trans-chrysanthémate de 1-éthynyl-2-méthylpenta-2-ène-1-yle, 8 g de N-(2-éthylhexyl)-1-isopropyl-4-méthylbicyclo[2,2,2]octo-5-ène-2,3-dicarboxy imide, 1 g de DBH, 0,8 g de parfum, 0,2 g de 1,4-diisopropyl aminoanthroquinone, et 10 g de kérosène désodorisé et en chauffant le mélange obtenu pour faciliter la dissolution des solides. On imprègne la même plaque de fibres comprimées que celle utilisée dans l'exemple 1 de 0,3 g de la solution et on la traite de la même façon pour produire un tampon feutré insecticide. Pour l'utilisation, ce tampon feutré est monté sur la plaque irradiant de la chaleur du fumigateur thermique représenté sur les figures 1 et 2. Ce fumigateur est essayé sur des moustiques et se révèle présenter le même effet que n'importe quel serpentin à moustiques du commerce pendant plus de dix heures.

## Exemple 4

Une plaque de céramique de 30 x 20 x 3 mm est imprégnée de 2 ml de solution éthanolique de 1 g de dioxine. Pour l'utilisation, la plaque de céramique mouillée est montée sur la plaque irradiant de la chaleur du fumigateur thermique représenté sur les figures 1 et 2 pour effectuer une fumigation dans une pièce. Les nombres de germes dans la pièce avant et après la fumigation ont été comparés par la méthode de la boîte de Pétri en utilisant un milieu de culture de gélose. On trouve que la fumigation a réduit le nombre de germes à moins de 1 %.

## Exemple 5

On prépare un gel en dissolvant 1 g de polymère carboxyvinylique, (produit vendu sous la marque commerciale High-bis Waco 104) dans 47 g d'une solution éthanolique contenant 5 g de parfum, puis on en ajoutant 2 g d'une solution aqueuse à 2 % de triéthanolamine à la solution obtenue. Dans un récipient d'aluminium, on introduit 20 g du gel, on monte le récipient sur la plaque irradiant de la chaleur du fumigateur thermique représenté sur les figures 1 et 2 et on l'utilise comme parfum d'ambiance.

## Exemple 6

La même plaque de fibres comprimées que celle de l'exemple 1 est imprégnée de 1 ml d'une solution alcoolique de 0,5 g d'extrait alcoolique du principe désodorisant présent dans les feuilles vivantes d'un camélia de la famille des Théacées. On le monte sur la plaque irradiant de la chaleur du fumigateur thermique représenté sur les figures 1 et 2 et on l'utilise dans un cabinet d'aisance pour essayer son effet désodorisant. La fumigation conduit à une élimination poussée de l'odeur agressive du cabinet.

La présente invention sera décrite à présent ci-dessous en se référant à des essais.

## Essai 1:

On soumet le fumigateur thermique représenté sur les figures 1 et 2 à un essai d'effet insecticide en fonction du temps sur des moustiques, avec les tampons feutrés insecticides obtenus dans les exemples 1 et 2, montés chacun sur la plaque irradiant de la chaleur et 25 g d'un gel préparé à partir de 90 parties de méthanol, 8 parties d'éthanol et 8 parties d'un dérivé benzylidène du D-sorbitol (vendu sous la marque commerciale Gelol D) placé sur le récipient à combustible. Les résultats sont donnés à la figure 8. Le terme d'"effet relatif" utilisé dans la figure désigne la variation d'effet du produit de fumigation telle que déterminée à des intervalles d'une heure et exprimée par rapport à l'effet déterminé au bout de la première heure considéré comme égal à 1,0. Il ressort clairement de la figure 10 que les tampons feutrés insecticides des exemples 1 et 2 présentent des effets insecticides élevés.

## Essai 2:

Dans le fumigateur thermique représenté sur les figures 1 et 2, la température de l'élément irradiant de la chaleur 4 est mesuré tandis que l'on fait varier d'un essai à l'autre la distance (C cm) entre le catalyseur 5 et la plaque irradiant de la chaleur 41 et la distance (d cm) entre le catalyseur 5 et le récipient à combustible 8 rempli

9

du combustible jusqu'à l'extrémité supérieure de son ouverture et le passage du gaz est maintenu ouvert dans certains essais et fermé dans d'autres essais. Les résultats sont donnés dans le tableau 1. Dans cet essai, un combustible solide préparé en dissolvant à chaud six parties d'acide stéarique dans 86 parties de méthanol et en ajoutant à la solution obtenue 8 parties d'une solution aqueuse d'hydroxyde de sodium à 12,5 % (eau : méthanol = 1 : 8) est utilisé comme combustible pour le fumigateur. La température de la pièce dans lequel l'essai est effectué est maintenue à 25°C ± 1°C.

**Tableau 1**

| Essai | Distance (C cm) | Distance (d cm) | Utilisation d'un passage à gaz | Température moyenne de la plaque plaque irradiant de la chaleur* (°C) |
|---|---|---|---|---|
| 1 | 0,3 | 0 | oui | 57 |
| 2 | 0,3 | 0,2 | oui | 67 |
| 3 | 0,3 | 0,3 | oui | 120 |
| 4 | 0,3 | 0,7 | oui | 140 |
| 5 | 0,3 | 1,0 | oui | 140 |
| 6 | 0,3 | 3,0 | oui | 143 |
| 7 | 0,3 | 5,0 | oui | 125 |
| 8 | 0,3 | 7,0 | oui | 100 |
| 9 | 0,3 | 10,0 | oui | 86 |
| 10 | 0,3 | 12,0 | oui | 64 |
| 11 | 0 | 1,0 | oui | 63 |
| 12 | 0,1 | 1,0 | oui | 72 |
| 13 | 0,2 | 1,0 | oui | 119 |
| 14 | 0,5 | 1,0 | oui | 134 |
| 15 | 1,0 | 1,0 | oui | 113 |
| 16 | 3,0 | 1,0 | oui | 91 |
| 17 | 4,0 | 1,0 | oui | 70 |
| 18 | 0,3 | 1,0 | non | 54 |

* L'expression "température moyenne" désigne la moyenne des température mesurées toutes les heures jusqu'à un total de dix heures.

Les résultats du tableau 1 indiquent les faits suivants.

La comparaison des résultats de l'essai no. 1 et de ceux des essais nos 2 à 10 révèle que l'interposition d'une distance entre le récipient à combustible (combustible) 8 et le catalyseur 5 est nécessaire et que la température de l'élément irradiant de la chaleur 4 peut être réglée par cette distance. L'interposition d'une distance entre le catalyseur 5 et la plaque irradiant de la chaleur 41 se révèle également nécessaire à partir de la comparaison des résultats des essais Nos 5 et 11. En comparant les résultats de l'essai no. 5 et ceux des essais Nos 11 à 17, on voit que cette distance (c cm) affecte la température de la plaque irradiant de la chaleur 41. Ceci signifie qu'une combinaison appropriée des distances, c cm et d cm, permet de choisir la température de la plaque irradiant de la chaleur 41, en fonction de la nature de la drogue et du but de la fumigation. La comparaison des résultats de l'essai no. 4 et de ceux de l'essai no. 18 révèle que la présence d'un passage de gaz est indispensable pour le fonctionnement du fumigateur. Un essai séparé effectué en ce qui concerne la taille et le nombre des passages de gaz formés dans le fumigateur a donné des résultats qui indiquent qu'une absence absolue d'une élévation de température de la plaque irradiant de la chaleur ne peut pas se produire en présence d'un passage de gaz et que la température de la plaque irradiant de la chaleur est fixée lorsque la taille du passage de gaz est fixée. Les résultats montrent également que le passage de gaz ne doit pas nécessairement être formé dans la plaque de dessus 21 du couvercle du boîtier 2 et que l'effet de la présence d'un passage de gaz est le même lorsque le passage de gaz est formé dans la paroi latérale 27 du couvercle de boîtier 2 et lorsqu'il est formé dans la paroi externe 1d du corps de boîtier 1.

Les résultats indiquent aussi que la taille de l'ouverture pratiquée dans le côté supérieur du récipient à carburant varie avec la nature du carburant effectivement utilisé et la distance entre le récipient à combustible et le catalyseur, mais que la température de la plaque irradiant de la chaleur est stable lorsque toutes ces conditions sont fixées.

A partir des résultats de l'essai décrit ci-dessus, on note que pour qu'une plaque irradiant de la chaleur dans le fumigateur thermique de la présente invention soit chauffée efficacement avec une source de chaleur donnée, il est impératif d'interposer une distance déterminée entre le récipient à combustible et le catalyseur et une distance déterminée entre le catalyseur et la plaque irradiant de la chaleur et en outre de munir le fumigateur d'un passage de gaz et que les autres conditions du fumigateur peuvent être modifiées de manière appropriée en fonction du but de la fumigation, de la nature de la drogue et du combustible, etc.

## 0 120 968

**Essai 3:**

Dans le fumigateur thermique des figures 1 et 2, et dans celui des figures 4 et 5, on mesure les températures des plaques irradiant de la chaleur respectives tout en faisant varier la nature du carburant volatil utilisé d'un essai à l'autre. La température de la pièce dans laquelle l'essai est effectué est maintenue à 25 ± 1°C.

| Essai No. | Combustible volatil | Température moyenne de la plaque irradiant de la chaleur* (°C) |
|---|---|---|
| 1 | Méthanol | 141 |
| 2 | Combustible solide constitué de méthanol | 145 |
| 3 | Combustible solide constitué de méthanol et d'éthanol | 139 |
| 4 | Hexane | 136 |
| 5 | Benzène | 140 |
| 6 | Gaz de pétrole liquéfié | 144 |
| 7 | Ether diméthylique | 140 |
| 8 | Gaz pour briquet | 137 |

\* L'expression "température moyenne" désigne la moyenne des températures mesurées toutes les heures jusqu'à un total de dix heures.

Compte tenu des résultats de l'essai 3 décrit ci-dessus, on note que dans le fumigateur thermique de la présente invention, la plaque irradiant de la chaleur atteint la température attendue dans la mesure où le combustible qui est utilisé possède une volatilité à la température ambiante et où la variation de la nature du combustible volatil ne présente pas de différence significative à cet égard.

Dans les essais Nos. 4 à 8, une brève application de chaleur au catalyseur au moment où le fumigateur est mis en service a pour effet d'augmenter la vitesse de dégagement de chaleur.

Comme il ressort de la description qui précède, le fumigateur thermique de la présente invention réalise la vaporisation thermique désirée d'une drogue donnée en plaçant dans le boîtier du fumigateur un combustible volatil ou un combustible de gaz liquéfié, en disposant un catalyseur au-dessus du carburant en travers d'un espace fixé, en permettant au carburant de subir une réaction d'oxydation dans le catalyseur, en permettant à la chaleur de cette réaction d'oxydation d'être transférée à une plaque irradiant de la chaleur disposée au-dessus du catalyseur en travers d'un espace fixé, et en amorçant une élévation de la température de la plaque irradiant de la chaleur, de sorte que la plaque irradiant de la chaleur chauffe la drogue déposée sur elle. Par comparaison avec le fumigateur à drogues classique, qui utilise de l'électricité comme source de chaleur pour la vaporisation d'une drogue, le fumigateur thermique de la présente invention présente l'avantage que sa construction est simple et qu'il n'est pas limité par l'emplacement de fonctionnement. Etant donné que la plaque irradiant de la chaleur est chauffée uniformément par oxydation du combustible en présence du catalyseur et que, par conséquent, la drogue est vaporisée uniformément, la drogue incorporée par imprégnation d'un tampon feutré de fibres comprimées peut être évaporée de manière régulière à partir de ce tampon feutré. Ce fumigateur présente l'avantage remarquable de permettre la conservation uniforme de la température disponible, considérée jusqu'à présent comme difficilement réalisable avec le fumigateur classique.

En particulier, le fumigateur thermique de la présente invention permet d'utiliser un gaz liquéfié comme combustible capable de donner lieu à une réaction d'oxydation en présence d'un catalyseur métallique, et en conséquence se révèle extrêmement commode en ce qu'il permet de recharger librement le gaz liquéfié et de réguler librement la libération du combustible. Il est ainsi extrêmement portable.

## Revendications

1. Fumigateur thermique de drogues, telles que des insecticides, des fongicides, des parfums d'ambiance et des déodorants, comportant un boîtier (1) avec un réceptacle à combustible (8 ; 8') contenant un combustible volatil ou un gaz liquéfié apte à réagir avec un catalyseur métallique (5) disposé de façon séparée au-dessus du réceptacle à combustible, une plaque (41) irradiant de la chaleur, pour effectuer la vaporisation thermique de la drogue (3), étant disposée au-dessus du catalyseur métallique pour supporter l'élément insecticide à vaporiser, et le boîtier (1) étant muni de passages pour l'entrée de l'air ambiant et l'évacuation des gaz de combustion et des vapeurs de la drogue (23, 24, Figures 1, 2 et 4 ; 23' et 23, Figure 5), caractérisé en ce qu'un espace (D) est ménagé entre le catalyseur 5 et le point de sortie des vapeurs ou gaz combustible hors du réceptacle à combustible (8 ; 8') et en ce qu'un autre espace (C) est laissé libre entre la plaque (41) et le catalyseur métallique (5).

2. Fumigateur thermique selon la revendication 1, caractérisé en ce que le catalyseur métallique est porté par une plaque d'isolation thermique (6) disposée au-dessus du réceptacle à combustible (8 ; 8').

11

3. Fumigateur thermique selon la revendication 1, caractérisé en ce que le catalyseur métallique (5) et la plaque irradiant de la chaleur (41) sont réunis par un élément métallique (42) délimitant l'espace (C).

4. Fumigateur thermique selon l'une quelconque des revendications 1 à 3, caractérisé en ce que le point de sortie des vapeurs ou gaz combustible du réceptacle à combustible (8) et le catalyseur métallique (5) sont séparés l'un de l'autre par une distance (D) de 0,3 à 10 cm.

5. Fumigateur thermique selon l'une quelconque des revendications 1 à 4, caractérisé en ce que la distance (C) séparant le catalyseur métallique (5) et la plaque irradiant de la chaleur (41) est comprise entre 0,2 et 3 cm.

6. Fumigateur thermique selon la revendication 1, caractérisé en ce que le boîtier (1) renferme un récipient contenant un gaz liquéfié, une buse (65) communiquant avec ce récipient par une soupape (65), un catalyseur métallique (5) disposé dans une position dans laquelle le gaz liquéfié issu de la buse (65) rencontre le catalyseur métallique (5) et une plaque irradiant de la chaleur (41) pour la vaporisation thermique d'une drogue (3) disposée au voisinage de ce catalyseur métallique, et en ce que le boîtier (1) est muni de passages (23 ; 23') pour l'entrée d'air et l'évacuation du gaz de combustion et est en outre muni d'un système de commande de soupape (B - Fig. 5) capable de réguler l'ouverture et la fermeture de cette soupape.

7. Fumigateur thermique selon la revendication 6, caractérisé en ce que le catalyseur métallique (5) est disposé au-dessus de la buse (65), la plaque irradiant de la chaleur (41) pour la vaporisation thermique d'une drogue étant disposée au-dessus de ce catalyseur métallique.

## Patentansprüche

1. Vorrichtung zur Heißverflüchtigung von Agenzien, wie Insektiziden, Fungiziden, in die Umgebung abzugebenden Duftstoffen und Deodoranten, mit einem Gehäuse (1) mit einem Brennstoffbehälter (8; 8'), der einen sich verflüchtigenden Brennstoff oder ein Flüssiggas aufweist, wobei der Brennstoff bzw. das Gas geeignet ist, mit einem metallischen Katalysator (5) zu reagieren, der gesondert davon oberhalb des Brennstoffbehälters angeordnet ist, und mit einer zwecks thermischer Verdampfung des Agens (3) vorgesehenen Wärmestrahlungsplatte (41), die oberhalb des metallischen Katalysators zur Aufnahme des zu verflüchtigenden Insektizid-Elementes angeordnet ist, und mit in dem Gehäuse (1) vorgesehenen, zum Eintritt von Umgebungsluft und zur Abfuhr der Verbrennungsgase und der Dämpfe des Agens vorgesehenen Durchgangsöffnungen (23, 24, Figuren 1, 2 und 4; 23' und 23, Fig. 5), dadurch gekennzeichnet, daß zwischen dem Katalysator (5) und der Austrittsstelle der Dämpfe oder des Brenngases aus dem Brennstoffbehälter (8; 8') ein Zwischenraum (D) vorgesehen ist, und daß zwischen der Platte (41) und dem metallischen Katalysator (5) ein weiterer Zwischenraum (C) vorgesehen ist.

2. Vorrichtung zur Heißverflüchtigung nach Anspruch 1, dadurch gekennzeichnet, daß der metallische Katalysator von einer oberhalb des Brennstoffbehälters (8; 8') angeordneten Wärmeisolationsplatte (6) getragen ist.

3. Vorrichtung zur Heißverflüchtigung nach Anspruch 1, dadurch gekennzeichnet, daß der metallische Katalysator (5) und die Wärmestrahlungsplatte (41) über ein den Zwischenraum (C) begrenzendes metallisches Element (42) verbunden sind.

4. Vorrichtung zur Heißverflüchtigung nach einem der Ansprüche 1 bis 3, dadurch gekennzeichnet, daß die Austrittsstelle der Dämpfe oder des Brenngases aus dem Brennstoffbehälter (8) und der metallische Katalysator (5) in einem gegenseitigen Abstand (D) zwischen 0,3 bis 10 cm voneinander getrennt angeordnet sind.

5. Vorrichtung zur Heißverflüchtigung nach einem der Ansprüche 1 bis 4, dadurch gekennzeichnet, daß der Abstand (C) zwischen dem metallischen Katalysator (5) und der Wärmestrahlungsplatte (41) zwischen 0,3 und 3 cm beträgt.

6. Vorrichtung zur Heißverflüchtigung nach Anspruch 1, dadurch gekennzeichnet, daß das Gehäuse (1) einen Behälter mit Flüssiggas, eine mit diesem Behälter über ein Ventil (65) verbundene Düse (65), einen in einer Lage, in der das aus der Düse (65) austretende Flüssiggas auf den metallischen Katalysator (5) auftrifft, angeordneten metallischen Katalysator und eine Wärmestrahlungsplatte (41) zur thermischen Verflüchtigung eines dem metallischen Katalysator benachbart angeordneten Agens (3) aufweist, und daß das Gehäuse (1) Durchgangsöffnungen (23; 23') zum Eintritt von Luft und zur Abfuhr von Verbrennungsgas aufweist, und daß darüber hinaus ein zur Steuerung des Öffnens und des Schließens dieses Ventils geeignetes Ventilsteuerungssystem (B - Fig. 5) vorgesehen ist.

7. Vorrichtung zur Heißverflüchtigung nach Anspruch 6, dadurch gekennzeichnet, daß der metallische Katalysator (5) oberhalb des Brenners (65) angeordnet ist, und daß die Wärmestrahlungsplatte (41) für die thermische Verflüchtigung eines Agens oberhalb dieses metallischen Katalysators angeordnet ist.

## Claims

1. Thermal fumigator for drugs such as insecticides, fungicides, room-scenting perfumes and deodorants, comprising a casing (1) with a fuel receptacle (8 ; 8') containing a volatile fuel or of liquefied gas which is capable of reacting with a metallic catalyst (5) placed separately above the fuel receptacle, a heat-irradiating plate (41) for carrying out thermal vaporization of the drug (3) being placed above the metallic catalyst for supporting the insecticide element to be vaporized, and the casing (1) being provided with passages for admission of ambient air and discharge of combustion gases and vapours of the drug (23, 24, Figures 1, 2 and 4; 23' and 23, Figure 5), characterized in that a space (D) is arranged between the catalyst 5 and the point of discharge of the vapours or fuel gas from the fuel receptacle (8 ; 8') and that another space (C) is left free between the plate (41) and the metallic catalyst (5).

2. Thermal fumigator in accordance with claim 1, characterized in that the metallic catalyst is carried by a heat-insulating plate (6) placed above the fuel receptacle (8 ; 8').

3. Thermal fumigator in accordance with claim 1, characterized in that the metallic catalyst (5) and the heat-irradiating plate (41) are connected to each other by means of a metallic element (42) which delimits the space (C).

4. Thermal fumigator in accordance with any one of claims 1 to 3, characterized in that the point of discharge of the vapours or fuel gas from the fuel receptacle (8) and the metallic catalyst (5) are spaced apart at a distance (D) of 0.3 to 10 cm.

5. Thermal fumigator in accordance with any one of claims 1 to 4, characterized in that the distance (C) between the metallic catalyst (5) and the heat-irradiating plate (41) is within the range of 0.2 to 3 cm.

6. Thermal fumigator in accordance with claim 1, characterized in that the casing (1) encloses a vessel containing a liquefied gas, a nozzle (65) communicating with this vessel via a valve (65), a metallic catalyst (5) placed in a position in which the liquefied gas issuing from the nozzle (65) encounters the metallic catalyst (5) and a heat-irradiating plate (41) for thermal vaporization of a drug (3) placed in the vicinity of this metallic catalyst, and that the casing (1) is provided with passages (23 ; 23') for admission of air and discharge of the combustion gas and is additionally provided with a valve control system (B - Fig. 5) which is capable of regulating the opening and closing of this valve.

7. Thermal fumigator in accordance with claim 6, characterized in that the metallic catalyst (5) is placed above the nozzle (65), the heat-irradiating plate (41) for thermal vaporization of a drug being placed above this metallic catalyst.

0 120 968

# F I G. I

# F I G. 2

## FIG. 3 A

41

42

42

43

44lb

441

441a    44    441a

441

## FIG. 3 B

41

42

42

43    442    442b    44    442a    442    442a

442a    442b

442    443

3

FIG. 4

FIG. 5

# F I G. 6

## FIG. 7

## FIG. 8